# EUROPEAN PATENT APPLICATION

(11) **EP 4 070 792 A1**
(43) Date of publication of application: **12.10.2022**
(21) Application number: 20895330.7
(22) Date of filing: 04.12.2020
(51) Int. Cl.: A61K 31/335, A61K 9/08, A61K 9/12, A61K 47/02, A61K 47/04, A61K 47/14, A61K 47/18, A61K 47/32, A61P 11/02, A61P 27/02, A61P 37/08

(54) **RHINENCHYSIS COMPOSITION CONTAINING OLOPATADINE**

(30) Priority: 06.12.2019 JP 2019221420
(71) Applicant: Toko Yakuhin Kogyo Co., Ltd., Osaka-shi, Osaka 530-0022 (JP)
(72) Inventor: KAMISHITA, Taizou, Osaka-shi, Osaka 530-0022 (JP); MIYAZAKI, Takashi, Osaka-shi, Osaka 530-0022 (JP)
(74) Representative: Hoffmann Eitle
(86) International application number: PCT/JP2020/045312
(87) International publication number: WO 2021/112240

(57) **Abstract**

The present invention relates to a composition wherein olopatadine is stably dissolved and a process thereof.

## Description

### TECHNICAL FIELD

The present invention relates to a composition comprising olopatadine which is in stably soluble state, and a process thereof. The present invention may be also used for treating allergic rhinitis.

### BACKGROUND ART

Olopatadine has been already used as antihistamine in the treatment of allergic rhinitis or allergic conjunctivitis. Patent Reference 1 discloses a nasal drop comprising olopatadine hydrochloride, specifically an invention directed to a drug product comprising 0.54 - 0.62 % (w/v) olopatadine free base, wherein the pH is adjusted to 3.6 - 3.8, which is a topical formulation in use for treating and/or preventing nasal allergic or inflammatory disorder.

As a drug preparation based on the patent reference, PATANASE^{™} (olopatadine hydrochloride solution for nasal use) 0.6 % is an only commercial preparation for use in nasal administration, comprising olopatadine. According to the label information, the preparation comprises olopatadine hydrochloride whose amount is 0.6 % as olopatadine free form, 0.01 % benzalkonium chloride, and non-specified amounts of disodium hydrogen phosphate, disodium edetate hydrate, sodium chloride, hydrochloric acid and/or sodium hydroxide (for adjusting the pH), and purified water, but the pH is adjusted to about 3.7 which is a very irritant pH for intranasal mucosa.

A similar composition to PATANASE^{™} wherein the pH is adjusted to about 7 has been also marketed under tha name PATANOL^{™}, but the concentration of olopatadine in PATANOL^{™} is very low, i.e., 0.1 % as olopatadine free base, because the solubility of olopatadine is very low around neutral pH.

The poor solubility of olopatadine had been studied in detail in Pantent Reference 1, which includes two kinds of "figures showing pH-solubility profile of olopatadine", said figures show that olopatadine whose concentration is 0.2 % or more as olopatadine free base is not soluble in a solution having pH of 5 or higher. Thus, in order to make olopatadine whose concentration is 0.2 % or more in solution state, the solution must be adjusted to pH of 3.6 - 3.8. However, such pH was hardly physiologically-acceptable as eyedrop composition as well as nasal drop composition from the viewpoint of irritancy in nasal mucosa.

### PRIOR ART

### [Patent Reference]

[Patent Reference 1] JP 5149308 B

### SUMMARY OF INVENTION

### (Technical Problem)

The purpose of the present invention is to provide a pharmaceutical composition for use in nasal administration comprising olopatadine which is poorly-soluble in an aqueous solution at a pH range of very mild acidity to near neutrality. The pH in human adult nasal cavity varies between 5.5 - 6.5, and thus it is preferable that the pH of such aqueous solution does not greatly deviate from the pH in the nasal cavity since it is physiologically mildly-irritating, for example, pH 4.0 - 7.0, preferably 4.5 - 6.5, and more preferably 5.0 - 6.0. Thus, the pharmaceutical composition required for the present invention is a pharmaceutical composition comprising olopatadine, wherein olopatadine is soluble even in high concentrations at the above-mentioned pH range, which has a good retention in the nasal cavity after spray-administration.

### (Solution to Problem)

The present inventors have extensively studied on the above problem and have found that poorly-soluble olopatadine can be maintained in a stable solution state in high concentrations even at pH range of 5.0 - 6.0 by adding carboxy vinyl polymer to a nasal drop composition comprising olopatadine, said carboxy vinyl polymer is usually used as thickening agent or viscous agent, not used as solubilizer or solubilizing agent. Based upon the new findings, the present invention has been accomplished.

The present invention may provide the following embodiments.

(Item 1) A composition for use in nasal administration comprising olopatadine or a pharmaceutically acceptable salt thereof, and carboxy vinyl polymer, wherein the pH is adjusted to 4.0 - 7.0 (preferably 4.5 - 6.5, more preferably 5.0 - 6.0).

(Item 2) The composition for use in nasal administration of Item 1, wherein the olopatadine or a pharmaceutically acceptable salt thereof is olopatadine hydrochloride, and the olopatadine hydrochloride is in solution state in a concentration of 0.2 % (w/w) or more.

(Item 3) The composition for use in nasal administration of Item 1 or 2, wherein the concentration of the carboxy vinyl polymer is 0.1 - 2 % (w/w).

(Item 4) The composition for use in nasal administration of any one of Items 1 - 3, wherein the pH is adjusted with L-arginine and/or sodium hydroxide and/or hydrochloric acid as a pH adjuster.

(Item 5) The composition for use in nasal administration of any one of Items 1 - 4, further comprising one or more preservatives.

(Item 6) The composition for use in nasal administration of Item 5, wherein the preservative comprises benzalkonium chloride.

(Item 7) The composition for use in nasal administration of Item 5 or 6, wherein the preservative comprises disodium edetate hydrate.

(Item 8) The composition for use in nasal administration of any one of Items 1 - 7, further one or more isotonizing agents.

(Item 9) The composition for use in nasal administration of Item 8, wherein the isotonizing agent comprises sodium chloride and/or glycerin.

(Item 10) The composition for use in nasal administration of Item 8 or 9, wherein the concentration of the isotonizing agent is 0.1 - 10 % (w/w).

(Item 11) The composition for use in nasal administration of any one of Items 1 - 10, which is isotonic.

(Item 12) The composition for use in nasal administration of any one of Items 1 - 11, wherein the viscosity is 250 - 2500 mPa·s (preferably 500 - 1500 mPa·s).

(Item 13) The composition for use in nasal administration of any one of Items 1 - 12, whose mean liquid particle size is 30 - 100 µm when sprayed.

(Item 14) The composition for use in nasal administration of any one of Items 11 - 13, wherein the pH adjuster is L-arginine and sodium hydroxide, the preservative is disodium edetate hydrate and benzalkonium chloride, and the isotonizing agent is glycerin and sodium chloride.

(Item 15) A formulation for spray-administration to nasal cavity, comprising the composition for use in nasal administration of any one of Items 1 - 14.

(Item 16) A composition for use in nasal administration which is prepared by adding carboxy vinyl polymer to the composition to dissolve olopatadine at pH 5.0 - 6.0.

### (Effect of the Invention)

The present invention is expected to be an aqueous liquid comprising olopatadine or a pharmaceutically acceptable salt thereof which can be used in a spray-type nasal drop, wherein olopatadine is dissolved at physiologically-acceptable pH 5.0 - 6.0, which has a good retention in the nasal cavity after spray-administration, and thereby also has suspended pharmacological effect.

### DESCRIPTION OF EMBODIMENTS

Olopatadine is a compound of formula: 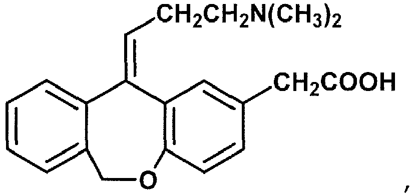 which has been already used as antihistamine for treating allergic rhinitis or allergic conjunctivitis.

Olopatadine or a salt thereof is used in the preparation of a composition for use in nasal administration, preferably used as olopatadine hydrochloride. The concentration of olopatadine in the present formulation is 0.2 % (w/w) or more, preferably 0.4 - 0.8 % (w/w), more preferably 0.4 - 0.7 % (w/w) as olopatadine hydrochloride.

The "pharmaceutically acceptable salt" is not limited to a specific one as long as the salt does not negatively affect humans or animals such as toxicity. It includes hydrochloride, citrate, succinate, hydrobromide, ascorbate, furoate, sulfate, acetate, valerate, oleinate, palmitate, laurate, stearate, bisulfate, borate, benzoate, lactate, phosphate, methanesulfonate, p-toluenesulfonate, oxalate, maleate, fumarate, tartrate, glucoheptonate, lactobionate, and laurylsulfate, as an acid-addition salt; and alkali metal salt such as sodium and potassium, and alkali-earth metal salt such as calcium and magnesium, as a basic salt.

Carboxy vinyl polymer used herein should not be limited as long as it is what is usually used in a medical formulation. Preferably, it is carboxy vinyl polymer whose viscosity is adjusted by adding an outside shearing force. The method of the adjustment and the effect of the modified carboxy vinyl polymer are disclosed in WO 2007/123193. For example, the outside shearing force may be added with a known device giving a shearing force such as a high-speed spinning-type emulsifying device, a colloidal mill-type emulsifying device, a high-pressure emulsifying device, a roll mill-type emulsifying device, an ultrasonic-type emulsifying device and a membrane-type emulsifying device. Especially, a homo mixer-type, a comb-type, and an intermittently-jet-stream-generating-type, high-speed spinning-type emulsifying devices are preferable. The content of carboxy vinyl polymer is 0.1 to 2 % (w/w), preferably 0.25 to 1.0 %.

The preservative herein includes, for example, benzalkonium chloride, benzethonium chloride, chlorobutanol and/or disodium edetate hydrate, preferably benzalkonium chloride and/or disodium edetate hydrate. The content of each preservative is 0.005 - 1 % (w/w), preferably 0.01 - 0.1 %.

The present aqueous composition for use in nasal administration is isotonic or around isotonic. The isotonicity may be adjusted with an isotonizing agent such as sodium chloride, boric acid, glycerin and/or glucose. The content of each isotonizing agent is 0.1 to 10 % (w/w), preferably 0.1 to 1.0 %.

The pH of the present aqueous composition for use in nasal administration needs to be adjusted to 4.0 - 7.0 (preferably 4.5 - 6.5, more preferably 5.0 - 6.0), and the adjustment of pH may be done with a pH adjuster such as sodium hydroxide, potassium hydroxide, L-arginine, and hydrochloric acid, preferably with sodium hydroxide and/or L-arginine.

The viscosity of the present aqueous composition for use in nasal administration is generally 250 - 2500 mPa·s, preferably 500 - 1500 mPa·s.

The "composition for use in nasal administration" used herein means an aqueous composition in soluble state, i.e., solution.

The "solution state" in the present invention means a state wherein an objective pharmaceutical ingredient is completely dissolved, and the "dispersion state" means a state wherein an objective pharmaceutical ingredient is homogeneously suspended without depositing crystal.

The liquid particle size of the present aqueous composition for use in. nasal administration means the particle size of the sprayed liquid particle. The mean liquid particle size is preferably 30 - 100 µm, more preferably 40 - 80 µm.

The "for use in nasal administration" or "as nasal drop" means a subject to be administered into the nasal cavity by dropping or with a device such as a spray device. And, the "composition for use in nasal administration" means a liquid preparation to be administered by dropping or spraying the composition into nasal cavity.

The nasal-spray preparation for intranasal administration of the present invention is directed to the nasal-spray preparation in a normal nasal spray container, or in an upper-pressure-relief airless-type spray container disclosed in WO 2007/123193 and WO 2007/123207.

### EXAMPLES

Hereinafter, the present invention is illustrated based on Examples, Reference examples, and Solubility test, but are not limited thereto. The evaluations of the Examples and Reference examples prepared below, and the solubility test and stability test were carried out according to Japanese Pharmacopoeia, unless otherwise indicated.

The viscosity measurement was carried out according to Japanese Pharmacopoeia/General Tests/Viscosity Determination/Viscosity measurement by rotational viscometer, and the details are as follows.

### (Measuring method)

1.1 mL of the test sample (test preparation) was charged into a sample cup of a cone-flat plate-type rotational viscometer (cone plate type) which was beforehand set for 20°C, while being careful not to put air bubble. The sample was let stand for 5 minutes, and then subjected to a shearing force for 3 minutes. Subsequently, the viscosity of the sample was measured according to the following condition.

### (Measuring condition)

Apparatus: TOKI SANGYO CO.,LTD. TVE-25 type viscosity meter
Measuring range: R (full-scale torque 1437.4 µN·m)
Shearing rate: 9.575 s⁻¹ (2.5 rotations per minute)
Rotor: 1°34'×R24

The liquid particle size (mean liquid particle size, 10 to 100 µm (%)) was measured with a laser diffraction/scattering particle-size-distribution analyzer.

### (Measuring condition)

Apparatus: Malvern SprayTec
Reading distance: 30 mm
Spray angle: 40°
Extrusion speed: 100 mm/s

### Example 1

### (Production composition)

| Ingredients | Amount (% by weight) |
|---|---|
| olopatadine hydrochloride | 0.443 |
| carboxy vinyl polymer | 0.50 |
| L-arginine | 0.73 |
| disodium edetate hydrate | 0.05 |
| benzalkonium chloride | 0.02 |
| concentrated glycerin | 0.50 |
| sodium hydroxide | q.s. |
| hydrochloric acid | q.s. |
| purified water | q.s. (until reaching 100 %) |
| Total | 100.0 |

### (Production method)

A solution of L-arginine, disodium edatate hydrate, concentrated glycerin, and olopatadine hydrochloride in purified water was charged into a vacuum mixer, then a solution of benzalkonium chloride in purified water was added thereto, and the mixture was stirred. Separately, carboxy vinyl polymer was dissolved in purified water with stirring and the solution was added to the mixture in the vacuum mixer. The mixture was stirred in the vacuum mixer. The pH of the mixture was adjusted to pH 5.5 optionally by adding aqueous sodium hydroxide or diluted hydrochloric acid, and then the mixture was subjected to a high-speed shearing force to adjust the viscosity to 1150 mPa·s with stirring.

### (Evaluation result)

The evaluation results of the obtained nasal composition are shown below.

| Aspect | A clear, colorless, viscous liquid, which is practically odorless |
|---|---|
| pH | 5.5 |
| viscosity (mPa·s) | 1150 |
| osmolality (mOs/L) | 276 |
| Mean liquid particle size (µm) | 69.4 |
| Liquid particle size of 10 to 100 µm (%) | 86.5 |

### Example 2

### (Production composition)

| Ingredients | Amount (% by weight) |
|---|---|
| olopatadine hydrochloride | 0.665 |
| carboxy vinyl polymer | 0.50 |
| L-arginine | 0.84 |
| disodium edetate hydrate | 0.05 |
| benzalkonium chloride | 0.01 |
| concentrated glycerin | 0.35 |
| ethanol | 0.8 |
| sodium hydroxide | q.s. |
| hydrochloric acid | q.s. |
| purified water | q.s. (until reaching 100 %) |
| Total | 100.0 |

### (Production method)

A solution of L-arginine, disodium edatate hydrate, concentrated glycerin, and olopatadine hydrochloride in purified water was charged into a vacuum mixer, then a solution of benzalkonium chloride in purified water was added thereto, and the mixture was stirred. Separately, carboxy vinyl polymer was dissolved in purified water with stirring and the solution was added to the mixture in the vacuum mixer. The mixture was stirred in the vacuum mixer. The pH of the mixture was adjusted to pH 5.5 optionally by adding aqueous sodium hydroxide or diluted hydrochloric acid, and then the mixture was subjected to a high-speed shearing force to adjust the viscosity to 1000 mPa·s with stirring. Finally, ethanol was added to the mixture and the mixture was stirred to give a uniform nasal composition.

### (Evaluation result)

The evaluation results of the obtained nasal composition are shown below.

| Aspect | A clear, colorless, viscous liquid, which is practically odorless |
|---|---|
| pH | 5.5 |
| viscosity (mPa·s) | 1000 |
| osmolality (mOs/L) | 281 |
| Mean liquid particle size (µm) | 62.2 |
| Liquid particle size of 10 to 100 µm (%) | 89.7 |

### Reference example 1

(According to Example 1 in JP 5149308 B)

### (Production composition)

| Ingredients | Amount (% by weight) |
|---|---|
| olopatadine hydrochloride | 0.665 |
| benzalkonium chloride | 0.01 |
| disodium edetate hydrate | 0.01 |
| sodium chloride | 0.41 |
| disodium hydrogen phosphate anhydrous | 0.5 |
| sodium hydroxide | q.s. |
| hydrochloric acid | q.s. |
| purified water | q.s. (until reaching 100 %) |
| Total | 100.0 |

### (Production method)

To purified water were added disodium hydrogen phosphate anhydrous, sodium chloride, disodium edetate hydrate, benzalkonium chloride, and olopatadine hydrochloride, while stirring. In order to dissolve each ingredient, appropriate quantities of diluted hydrochloric acid were added thereto with a moderate time interval. Purified water was added thereto, the pH was measured, the pH was adjusted to pH 3.7 optionally by adding hydrochloric acid or sodium hydroxide, and then purified water was added thereto to adjust the total weight.

### (Evaluation result)

The evaluation results of the obtained nasal preparation are shown below.

| Aspect | A clear, colorless liquid |
|---|---|
| pH | 3.7 |
| viscosity (mPa·s) | 3 |
| osmolality (mOs/L) | 278 |
| Mean liquid particle size (µm) | 51.8 |
| Liquid particle size of 10 to 100 µm (%) | 91.4 |

### Reference example 2

### (Production composition)

| Ingredients | Amount (% by weight) |
|---|---|
| olopatadine hydrochloride | 0.665 |
| benzalkonium chloride | 0.01 |
| disodium edetate hydrate | 0.01 |
| sodium chloride | 0.41 |
| disodium hydrogen phosphate anhydrous | 0.5 |
| sodium hydroxide | q.s. |
| purified water | q.s. (until reaching 100 %) |
| Total | 100.0 |

### (Production method)

To purified water were added disodium hydrogen phosphate anhydrous, sodium chloride, disodium edetate hydrate, benzalkonium chloride, and olopatadine hydrochloride, while stirring. In order to dissolve each ingredient, the mixture was warmed to about 60°C and stirred for an adequate time. Purified water was added thereto, the pH was measured, the pH was adjusted to pH 5.0 by adding sodium hydroxide, and then purified water was added thereto to adjust the total weight.

### (Evaluation result)

The evaluation results of the obtained nasal preparation are shown below.

| Aspect | A clear, colorless liquid |
|---|---|
| pH | 5.5 |
| viscosity (mPa·s) | 3 |
| osmolality (mOs/L) | 279 |
| Mean liquid particle size (µm) | 60.4 |
| Liquid particle size of 10 to 100 µm (%) | 88.1 |

### Solubility-stability test of olopatadine

### Liquid sample for comparative test

(1) Patanase^{™} (Lot No.: 7FPS1A)(Concentration pH 3.8)
   The content liquid was taken out of the product container, and put into a glass container to be a sample.
(2) A liquid prepared by dissolving olopatadine hydrochloride in purified water, adjusting the concentration of olopatadine to 0.6 % (w/w), and then adjusting pH to 3.8 with L-arginine (stored in a glass container)
(3) A liquid prepared by dissolving olopatadine hydrochloride in purified water, adjusting the concentration of olopatadine to 0.6 % (w/w), and then adjusting pH to 3.8 with sodium hydroxide (stored in a glass container)
(4) A liquid prepared by dissolving olopatadine hydrochloride in purified water, adjusting the concentration of olopatadine to 0.6 % (w/w), and then adjusting pH to 5.5 with L-arginine (stored in a glass container)
(5) A liquid prepared by dissolving olopatadine hydrochloride in purified water, adjusting the concentration of olopatadine to 0.6 % (w/w), and then adjusting pH to 5.5 with sodium hydroxide (stored in a glass container)

### (Evaluation method)

Each sample of (1) - (5), as well as Examples 1 and 2, and Reference example 1 were put into a glass container to be each sample for solubility stability test. The observation was done grossly.
o: confirmed to be in soluble state (clear).
×: confirmed that a crystal was precipitated.

### (Result)

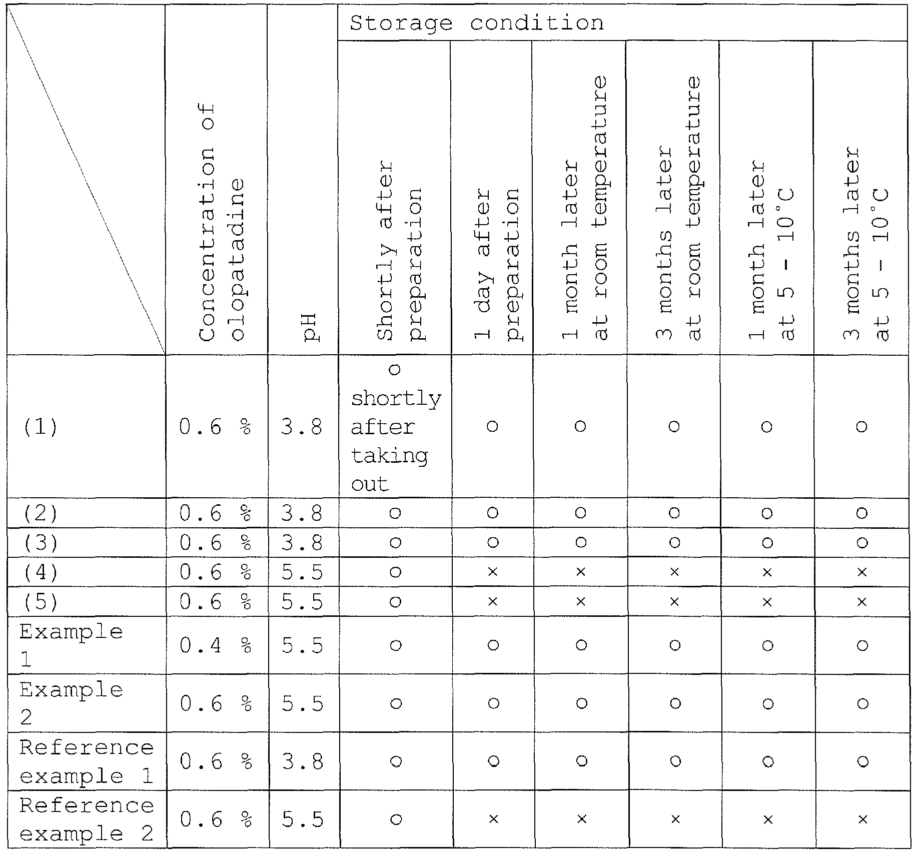

### INDUSTRIAL APPLICABILITY

The present invention is expected to be an aqueous spray-type nasal drop composition comprising olopatadine free base or an equivalent pharmaceutically acceptable salt thereof, wherein olopatadine is stably dissolved at physiologically-acceptable pH, which has a high viscocity and thus has a good retention in the nasal cavity after spray-administration, and thereby also has suspended pharmacological effect.

## Claims

1. A composition for use in nasal administration comprising olopatadine or a pharmaceutically acceptable salt thereof, and carboxy vinyl polymer, wherein the pH is adjusted to 4.0 - 7.0.

2. The composition for use in nasal administration of claim 1, wherein the olopatadine or a pharmaceutically acceptable salt thereof is olopatadine hydrochloride, and the olopatadine hydrochloride is in solution state in a concentration of 0.2 % (w/w) or more.

3. The composition for use in nasal administration of claim 1 or 2, wherein the concentration of the carboxy vinyl polymer is 0.1 - 2 % (w/w).

4. The composition for use in nasal administration of any one of claims 1 - 3, wherein the pH is adjusted with L-arginine and/or sodium hydroxide and/or hydrochloric acid as a pH adjuster.

5. The composition for use in nasal administration of any one of claims 1 - 4, further comprising one or more preservatives.

6. The composition for use in nasal administration of claim 5, wherein the preservative comprises benzalkonium chloride.

7. The composition for use in nasal administration of claim 5 or 6, wherein the preservative comprises disodium edetate hydrate.

8. The composition for use in nasal administration of any one of claims 1 - 7, further one or more isotonizing agents.

9. The composition for use in nasal administration of claim 8, wherein the isotonizing agent comprises sodium chloride and/or glycerin.

10. The composition for use in nasal administration of claim 8 or 9, wherein the concentration of the isotonizing agent is 0.1 - 10 % (w/w).

11. The composition for use in nasal administration of any one of claims 1 - 10, which is isotonic.

12. The composition for use in nasal administration of any one of claims 1 - 11, wherein the viscosity is 250 - 2500 mPa·s.

13. The composition for use in nasal administration of any one of claims 1 - 12, whose mean liquid particle size is 30 - 100 µm when sprayed.

14. The composition for use in nasal administration of any one of claims 11 - 13, wherein the pH adjuster is L-arginine and sodium hydroxide, the preservative is disodium edetate hydrate and benzalkonium chloride, and the isotonizing agent is glycerin and sodium chloride.

15. The composition for use in nasal administration of any one of claims 1 - 14, wherein the pH is adjusted to 4.5 - 6.5.

16. The composition for use in nasal administration of any one of claims 1 - 14, wherein the pH is adjusted to 5.0 - 6.0.

17. A formulation for spray-administration to nasal cavity, comprising the composition for use in nasal administration of any one of Items 1 - 16.

18. A composition for use in nasal administration which is prepared by adding carboxy vinyl polymer to the composition to dissolve olopatadine at pH 5.0 - 6.0.
